# EUROPEAN PATENT SPECIFICATION

(11) **EP 1 944 046 B1**
(45) Date of publication and mention of the grant of the patent: **11.08.2010**
(21) Application number: 07425010.1
(22) Date of filing: 12.01.2007
(51) Int. Cl.: A61M 1/34

(54) **Kit for treating a systematic inflammation related diseases**
Kit zur Behandlung von Erkrankungen im Zusammenhang mit systemischen Entzündungen
Kit pour le traitement de maladies liées à l'inflammation systémique

(43) Date of publication of application: 16.07.2008
(73) Proprietor: Bellco S.r.l., Mirandola (IT)
(72) Inventor: Atti, Mauro, 40131 Bologna (IT); Wratten, Mary Lou, 41036 Medolla (IT)
(74) Representative: Plebani, Rinaldo

(56) References cited:
- EP-A2- 0 958 839
- WO-A-03/009885
- WO-A-2006/002151
- US-A1- 2004 182 783

## Description

The present invention relates to a highly effective use of filters and sorbents for purifying blood in patients affected with systemic inflammatory related diseases.

Inflammation occurs as both a physiological and pathophysiological response to stress, such as injury, infection or a related specific disease, and results in local and general responses by the body. The local response is important for healing and as a defense against infection. This occurs via local production of specific and non-specific inflammatory mediators, such as angiopoietins, and cytokines. These are often involved in the systemic inflammatory response and the Systemic Inflammatory Response Syndrome (SIRS).

The general response takes place in the form of endocrinal, metabolic and biochemical reactions, with the extent of the response depending on the severity, intensity and duration of the stimulus. The general response is controlled by signals between the hypothalamic pituitary axis, the neuro-endocrinal hormone system and the autonomic nervous system. This coordinated action is referred to as the "stress response." The net effect of the stress response includes an increase in cardiac output, heart rate and blood pressure, peripheral and splanchnic vasoconstriction and coronary and cerebral vasodilation, increases in respiratory rate, sodium and water retention, increased coagulation, metabolic changes with hyperglycemia, and reduced urinary output.

While the stress response can be beneficial in aiding the recovery of the host, it is also a common link in many diverse critical illnesses. For instance, patients suffering from acute respiratory distress syndrome, acute lung injury, or acute respiratory failure who are on mechanical ventilation can experience trauma induced by the mechanical stretching of alveoli from the ventilator. The trauma can induce the subsequent release of inflammatory mediators, particularly vascular epithelial growth factor (VEGF), causing increased endothelial permeability, edema and increasing systemic inflammation and eventual organ dysfunction/failure. Patients with end stage renal diseases and diabetes also experience chronic inflammation and increased incidences of co-morbidities associated with it, such as cardiovascular disease. Vasculitis, a disease involving inflammation in blood vessels, can lead to damage of the body's organs, and even an aneurysm rupture. Patients suffering from sepsis and pancreatitis can also experience local and systemic inflammation over the course of the disease progression.

VEGF plays a role in a multitude of pathologies, including solid tumors and hematologic malignancies, intraocular neovascular syndromes, inflammation and brain edema, and pathology of the female reproductive tract (Ferrara et al., Nature Medicine, Vol. 9, No. 6, June 2003: 673-674). Current treatments to decrease VEGF are ranibizumab (LucentisTM, GenentechNovartis) pegaptanib (MacugenTM Pfizer / Eyetech) and Verteporfin PDT (Visudyne, Novartis) for age-related macular degeneration and bevacizubab (Genentech) for advanced colorectal cancer.

In patients suffering from the above-mentioned ailments, the blood gradually retains increasing quantities of toxins and inflammatory mediators. In healthy subjects, inflammatory mediators, cytokines or toxins are normally produced "as needed" and eliminated from the bloodstream. However, when the level of locally produced toxins rises uncontrollably, they can spill over into the plasma circulation causing profound endothelial dysfunction and the activation of many different types of inflammatory cells. This systemic inflammation and endothelial dysfunction can potentially lead to vascular permeability, organ hypoperfusion and eventual gut translocation of bacterial products such as endotoxin, which can further amplify the inflammatory response.

The process leading to multiorgan dysfunction is very complex and involves many overlapping pathways, including those of inflammation, coagulation as well as metabolic pathways. Pharmaceutical inactivation or immunomodulation of inflammatory mediators and cytokines is a generally known method for reducing blood toxins. However, it has been largely unsuccessful because inflammation involves redundant pathways. Additionally, inactivation of single mediators is often ineffective as other simultaneously produced mediators can still amplify the inflammatory response. Moreover, many mediators are produced after stimulating important pathways (such as NFkB).

Inactivation of such pathways can be detrimental if the same pathway also produces beneficial molecules. Finally, the detrimental effects of inflammatory mediators is often time dependent. Inactivation or removal of inflammatory mediators may be of benefit during mediator spill-over, but may be detrimental if the mediator plays a role in cell regeneration or healing. Unfortunately, many pharmaceuticals cannot be easily reversed or regulated.

Other commonly used methods of blood purification include absorbing the toxins on solid media (hemo- and plasmaperfusion), or by ultrafiltering the blood or plasma through appropriate semipermeable membranes, either by convection with the aid of a pressure gradient (TMP) through the membrane (hemo- or plasmafiltration), or by diffusion by bringing the blood or plasma to be purified into contact with one side of the membrane, and an appropriately formulated wash solution into contact with the opposite side (hemodialysis).

All of the above systems, however, present drawbacks. Hemoperfusion consists of percolating blood directly through a filter of adsorbent material, which must therefore be made highly biocompatible. This is usually achieved by covering the adsorbent particles with appropriate material which, however, seriously impairs the toxin-retaining capacity of the particles. In the case of plasmaperfusion, the blood is first filtered to separate the plasma, which is then percolated through the adsorbent material. Though this to some extent solves the problem of biocompatibility during the perfusion, the increase in the viscosity of the blood during filtration may result in extensive clotting through the membrane, so that in any case the blood must be treated with anticoagulants (heparin).

Hemo and plasmafiltration, on the other hand, only provide for removing high molecular weight toxins, and produce a considerable weight loss which must be compensated for by feeding an infusion solution into the patient's blood. According to EP 0958839B1, the above problem may be partly solved by regenerating the ultrafiltrate, by adsorbing the medium-high molecular weight toxins in it by percolating it through uncoated-activated-carbon-based hemoperfusion cartridges such as DETOXIL2™ (SORIN BIOMEDICA, Italy), so that the regenerated ultrafiltrate may be used, as it is or with additions, as an infusion solution.

Hemodialysis, particularly if combined with one or more of the above methods, is very effective in removing small water soluble toxins, but by itself, is largely ineffective for removing larger inflammatory mediators or toxins since these are not removed efficiently by diffusion. In particular, cytokine removal is fairly poor, so that, at present, organic malfunctions caused by acute organ failure can be no more than delayed as opposed to fully prevented.

This has been dealt with by EP0958839B1 at least in relation to a particular morbidity situation consisting in acute organ failure.

Also the arrangement shown in WO 2006/002151 does not solve the problem.

It is the aim of the present invention to provide an alternative means of treatment of systemic inflammation and of at least a number of its related diseases, which is more effective than the known solutions and allows different kind of toxins, especially low and middle molecular weight toxins, to be eliminated from the blood in a relatively short intervention time and that can be easily regulated in order to adapt it to individual patient conditions.

The present invention accordingly relates to a kit for treating a systemic inflammation related disease according to claim 1.

The goal of extracorporeal adsorption according to the present invention is to use a high permeable filter that allow passage of high molecular weight inflammatory mediators (not usually removed by conventional hemodialysis or hemofiltration filters which have smaller pore sizes), such as vascular endothelial growth factor (VEGF) and angiopoietins, as well serum albumin. The high permeable filter is thus associated to means for the retention of such inflammatory mediators by subsequent adsorption using an adsorbent cartridge with a high affinity for them. Differently from previously known methods, the means to retain the inflammatory mediators are selected in order not to retain serum albumin, which can be accordingly reinfused in the patient avoiding loss of one of the most important physiologic proteins necessary to maintain oncotic pressure, its antioxidant capacity and its function as a trasport protein for fatty acids, bilirubin, trypotphan, calcium, steroid hormones an many other physiologic compounds.

A further advantages of the use of a high permeability filter is the possibility to increase the blood flow to be purified compared to the normal plasma filters known in the art.

The high permeability filter has a pore size that ranges between 0.4 and 0.6 micron and anyway is such that to give rise to a sieving coefficient for the filter of less than 0.4 for IgM and of more than 0.6 for albumin.

The means to retain inflammatory mediators comprise at least one cartridge comprising a adsorbent material selected from the group consisting of a hydrophobic polystyrene resin, an ion-exchange polystyrene resin, a ultrapure bonded silica resin or mixtures thereof. Preferably, the hydrophobic polystyrene resins are chosen from the styrene-methylacrylate and copolymer divinylbenzene-polystyrene group of resins, of which the AMBERCHROM^{™} series of resin(Rohm Haas)is an example. The ultrapure silica resins are chosen from silica resins with bonded phase functional groups of which TSK Gel reverse phase resin (Tosoh Bioscience), such as ToyaPearl Phenyl-650 is an example. The ion-exchange resin is selected from the group consisting of DEAE Sepharose (Tosoh Bioscience) or Amberlite^{™} series of resin (Rohm Haas).

According to an embodiment of the invention the adsorbent material has a granules size comprised between 35 and 200 micron, and a pore size comprised between 50 and 3000 Å. Preferably, the cartridge comprises a polystyrene/divinylbenzene resin having a pore size of 300 Å and a granule size from 35 to 120 micron (for instance Rohm & Haas resin CG300™ grade S, M and C respectively). The most preferred one is resin CG300M having a mean diameter of the granules of from 75 to 120 micron.

In a preferred embodiment of the invention, the means to retain inflammatory mediators comprise more than one cartridge, each cartridge comprising a different adsorbent material designed to retain one or more different inflammation mediator(s), the inflammatory mediators retained by each cartridge being different from one another.

The inflammatory mediators which can be removed with the kit of the invention are selected in the group of VEGF, kallikrein, myoglobin, C-reactive protein, cytokines and chemokines (particularly IL1, IL6, IL8, IL12, IL18, Tumor necrosis factor, macrophage inflammatory protein-1, monocyte chemotactic protein).

The inventors surprisingly found that the association of a high permeable filter and of resins as those disclosed in EP 0958839B1, allows to retain inflammatory mediators other than cytokines, i.e. low -middle molecular weight mediators, without significant loss in serum albumin which can be thus reinfused in the patient. Moreover, as an additional consequence of this surprising discovery, the association of the above disclosed absorptive resins and high permeability filter allows them to be used to treat a fair large number of diseases not directly related to acute organ failure.

The inflammatory mediators retained (and so removed from the patient's blood stream) according to the present invention are associated generally to any systemic inflammation condition and more specifically to respiratory distress syndrome, acute lung injury, acute respiratory failure, severe pancreatitis, tumor lysis syndrome, myeloma, myasthenia gravis, vasculitis, rhabdomyolysis, systemic inflammatory response from coronary artery bypass grafting during cardiopulmonary bypass, systemic sclerosis, end stage renal diseases, age related macular degeneration, diabetic nephropathy.

Unlike more traditional methods of treating the above-mentioned illnesses, which include physical ingestion/exposure to drugs or irradiation which in essence are toxic to living systems, extracorporeal filtration has the advantage of the removal of toxins from the blood of the patient with minimal invasiveness. Additionally, removal can be done more quickly and for a specified time (duration) to remove mediators and then stopped when it is no longer necessary. This is advantageous over pharmacologic inhibition which often is not reversible and may require a longer duration of treatment.

Of added benefit is the adaptability of extracorporeal adsorption, whereby a wide array of nonspecific inflammatory mediators/cytokines can be tailored to an individual patient's needs (i.e., with add on cartridges). Moreover, with respect to other depurification techniques (such as high volume hemofiltration or plasma exchange), there can be selective removal of toxins or mediators but also reinfusion of physiologically important substances such as albumin, amino acids, and hormones.

Further aspects and advantages of the present invention will be apparent from the following description of several practical embodiments thereof given by way of non-limiting examples.

### Example 1 (not illustrating the Invention)

A high permeability plasmafilter is used which is made from the biocompatible material polyethersulfone. Additionally, a normal commercially available hemofilter is used. A cartridge containing 140 ml of divinylbenzene styrenic resin (Rohm Haas Amberchrom resin CG 300) with a pore size of 300 Å is used.

Table 1 shows the retention results obtained with different protein and mediators in human plasma samples of three septic patients.

**Table 1**

| | Monocyte chemotactic protein | Macrophage infiammatory protein 1β | metallo-proteinase-3 | Inter-leukin 6 IL-6 | Inter-leukin 8 IL8 | Inter-leukin 10 IL10 |
|---|---|---|---|---|---|---|
| | pg/ml | pg/ml | ng/ml | pg/ml | pg/ml | pg/ml |
| **Patient 1** | | | | | | |
| pre cartridge | 1330 | 408 | 28 | 270 | 437 | 269 |
| post cartridge | n.d. | n.d. | n.d. | n.d. | 5 | 11 |
| **Patient 2** | | | | | | |
| pre cartridge | 196 | 345 | 5.3 | 55 | 60 | 14 |
| post cartridge | 14 | 153 | n.d. | 9.5 | 54 | 0.8 |
| **Patient 3** | | | | | | |
| pre cartridge | 71 | 100 | 50 | 7.3 | 25 | 13 |
| post cartridge | n.d. | 4 | 3 | n.d. | 10 | 1.8 |
| | | | | | | |
| | n.d.= below level of detection | | | | | |

### Example 2

In vitro studies were done with human plasma containing added cytokines and mediators to determine affinities for different types of resins. Plasma was used to simulate the effluent of blood from a plasma filter having a sieving coefficient above 0.8 for human albumin. The flow of the blood would be between 100 and 200 ml/min while the flow of the plasma would be determined as a fractional filtration between 10 and 20% of the blood flow. The plasma filter would be used in series with a second filter for hemofiltration having a sieving coefficient below 0.1 for albumin (in order to remove small molecules not adsorbed by the resin or to maintain patient volume control). A cartridge containing 140 ml of divinylbenzene styrenic resin (Rohm Haas Amberchrom resin CG 300) with a pore size of 300 Å could be combined with other cartridges (listed in Table 2) in series for specific or nonspecific removal of inflammatory mediators, in particular, Interleukin 6 (IL-6), C-reactive protein (PCR) and Kallikrein (KK).

**Table 2**

| Resin No. | Resin | Particle size (µm) | Pore size (nm) |
|---|---|---|---|
| 1 | Toyopearl CM-650C | 100 | 100 |
| 2 | Toyopearl HW-40C | 75 | 5 |
| 3 | Toyopearl Mega CAP^{™} SP-550EC | 200 | 50 |
| 4 | Toyopearl SP-550-C | 100 | 50 |
| 5 | Toyopear135 Super SP | 40-90 | |
| 6 | CG71S | 35 | 250 |
| 7 | CG161M | 75 | 150 |
| 8 | CG300M | 75 | 300 |

5 ml of human plasma containing IL-6 (100 pg/ml), PCR (0.5 mg/dl) and KK (0.5 mg/l) for 4 hours was incubated with 1 ml resin. Samples were taken at 0, 60, 120, 180 and 240 minutes.

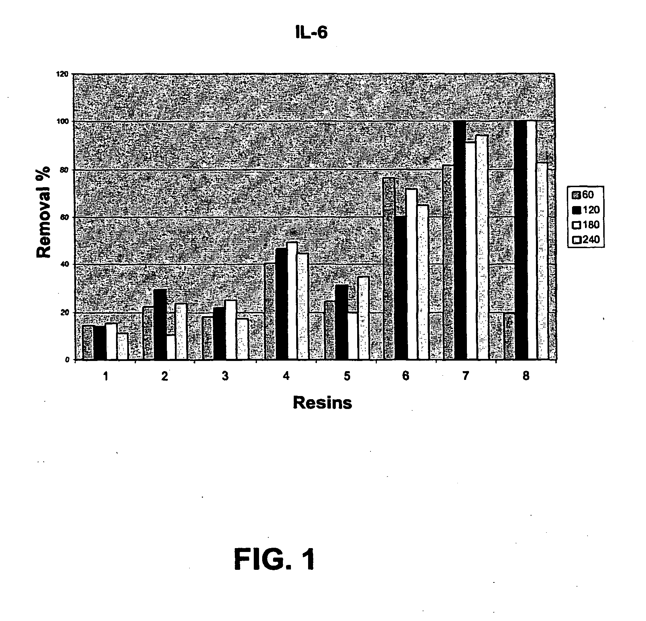

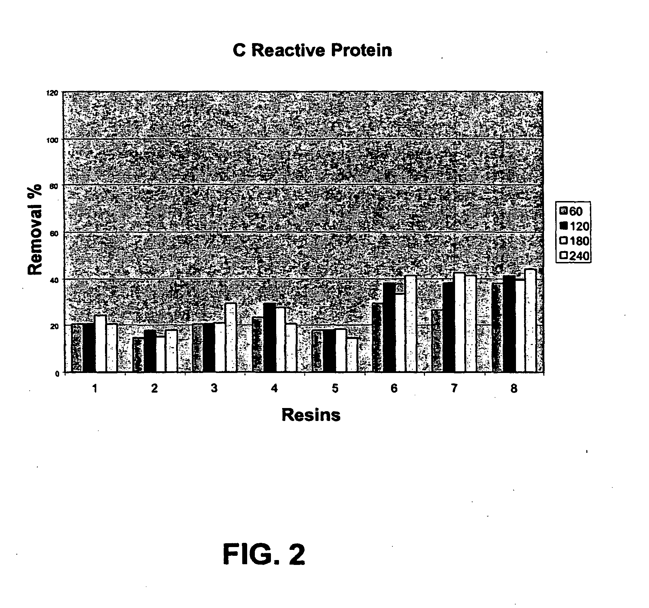

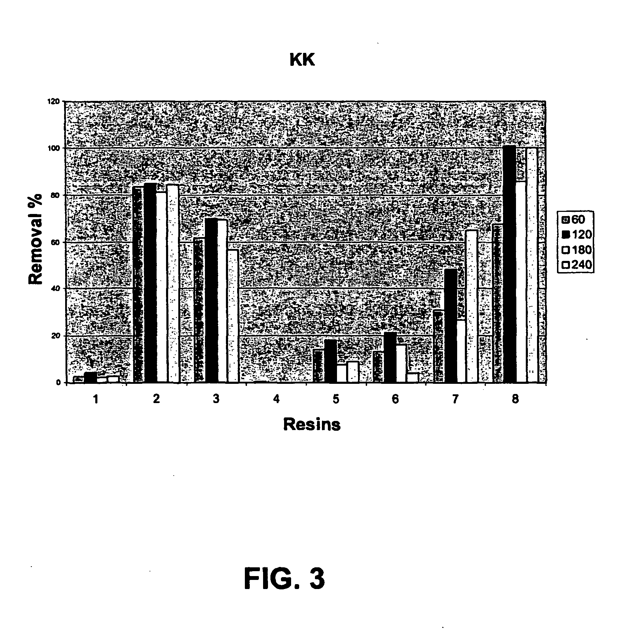

The obtained results are shown respectively in Figures 1, 2 and 3.

In particular, from Figure 1 it is evident that resins 6, 7 and 8 have a good affinity for IL-6. The same behavior is observed for PCR (Figure 2). On the contrary, KK is seen retained with resins 2, 3, 7 and 8 (Figure 3).

### Example 3

A high permeability plasmafilter, an hemofilter and a cartridge as in Example 1 are used.

Blood and plasma levels of VEGF are measured in 3 septic patients (normal ranges of VEGF are up to 55 pg/ml). Samples to determine VEGF amounts are taken at different time intervals; i.e., whole blood at time 0, plasma at 15 minutes prior to exposure to a filtration cartridge, and plasma at 15 minutes after exposure to a filtration cartridge. The results are expressed as VEGF pg/ml and are shown in Table 3.

**Table 3**

| | VEGF concentrations (pg/ml) | | |
|---|---|---|---|
| | Patient 1. | Patient 2 | Patient 3 |
| Blood (time 0) | 1490 | 1060 | 239 |
| Plasma 15' pre cartridge | 1720 | 708 | 233 |
| Plasma 15' post cartridge | 80 | 16 | 31 |

## Claims

1. Kit for treating a systemic inflammatory related disease comprising a) a high permeability filter having a pore size that ranges between 0.4 to 0.6 µm
to give rise to a sieving coefficient of the filter of less than 0.4 for IgM and of more than 0.6 for albumin
to let high molecular weight inflammatory mediators to pass and b) means to retain said mediators but not serum albumin comprising
at least one cartridge comprising a sorbent material selected from the group consisting of a hydrophobic polystyrene resin, an ion-exchange polystyrene resin, a bonded silica resin selected from the group consisting of silica resins with bonded phase functional groups, or mixtures thereof.

2. Kit according to claim 1, **characterized in that** said hydrophobic polystyrene resin is selected from the group consisting of the styrene-methylacrylate resins and copolymer divinylbenzene-polystyrene resins.

3. Kit according to claims 1 or 2, **characterized in that** said sorbent material has a granules size comprised between 35 and 200 micron.

4. Kit according to claims 1 or 2, **characterized in that** said adsorbent material has a pore size comprised between 50 and 3000 Å.

5. Kit according to any of the claims 1 to 3, **characterized in that** said cartridge comprises a polystyrene/divinylbenzene resin having a pore size of 300 Å and a granule size of from 35 to 120 micron.

6. Kit according to claim 5, **characterized in that** said cartridge comprises a polystyrene/divinylbenzene resin having a granule size of 75-120 micron.

7. Kit according to any of the preceding claims, **characterized in that** said means to retain inflammatory mediators comprise more than one cartridge, each cartridge comprising a different adsorbent material designed to retain one or more different inflammatory mediators, the inflammatory mediators retained by each cartridge being different from one another.

8. Kit according to claims 1 to 6, **characterized in that** said inflammatory mediators are selected from the group consisting of VEGF, Kallikrein, myoglobin, C-reactive protein, cytokines, and chemokines, in particular IL1, IL6, IL8, IL12, IL18, Tumor necrosis factor, macrophage inflammatory protein-1, monocyte chemotactic protein.

## Patentansprüche

1. Kit zur Behandlung einer systemischen entzündungsbedingten Erkrankung, umfassend a) einen hochpermeablen Filter mit einer Porengröße im Bereich von 0.4 bis 0.6 µm, was zu einem Siebkoeffizienten des Filters von weniger als 0.4 für IgM und von mehr als 0.6 für Albumin führt, so dass hochmolekulare Entzündungsmediatoren passieren können, und b) Mittel zum Zurückhalten der Mediatoren aber nicht von Serumalbumin, umfassend wenigstens eine Kassette, die ein Sorbensmaterial umfasst, ausgewählt aus der Gruppe bestehend aus einem hydrophoben Polystyrolharz, einem lonenaustausch-Polystyrolharz, einem derivatisierten Silicagel, ausgewählt aus der Gruppe bestehend aus Silicagelen mit trägergebundenen funktionellen Gruppen, oder Mischungen davon.

2. Kit nach Anspruch 1, **dadurch gekennzeichnet, dass** das hydrophobe Polystyrolharz ausgewählt ist aus der Gruppe bestehend aus Styrol-Methacrylat-Harzen und Divinylbenzol-Polystyrol-Copolymerharzen.

3. Kit nach Anspruch 1 oder 2, **dadurch gekennzeichnet, dass** das Sorbensmaterial eine Korngröße zwischen 35 und 200 Mikron hat.

4. Kit nach Anspruch 1 oder 2, **dadurch gekennzeichnet, dass** das Adsorbensmaterial eine Porengröße zwischen 50 und 3000 Å hat.

5. Kit nach einem der Ansprüche 1 bis 3, **dadurch gekennzeichnet, dass** die Kassette ein Polystyrol/Divinylbenzol-Harz mit einer Porengröße von 300 Å und einer Korngröße von 35 bis 120 Mikron umfasst.

6. Kit nach Anspruch 5, **dadurch gekennzeichnet, dass** die Kassette ein Polystyrol/Divinylbenzol-Harz mit einer Korngröße von 75-120 Mikron umfasst.

7. Kit nach einem der vorhergehenden Ansprüche, **dadurch gekennzeichnet, dass** die Mittel zum Zurückhalten von Entzündungsmediatoren mehr als eine Kassette umfassen, wobei jede Kassette ein anderes Adsorbensmaterial umfasst, das geeignet ist, ein oder mehr verschiedene Entzündungsmediatoren zurückzuhalten, wobei die Entzündungsmediatoren, die von jeder Kassette zurückgehalten werden, voneinander verschieden sind.

8. Kit nach Anspruch 1 bis 6, **dadurch gekennzeichnet, dass** die Entzündungsmediatoren ausgewählt sind aus der Gruppe bestehend aus VEGF, Kallikrein, Myoglobin, C-reaktivem Protein, Zytokinen und Chemokinen, insbesondere IL1, IL6, IL8, IL12, IL18, Tumornekrosefaktor, Makrophagen-Entzündungsprotein 1 und Monozyten-chemotaktischem Protein.

## Revendications

1. Kit pour le traitement d'une maladie liée à l'inflammation systémique comprenant a) un filtre hautement perméable ayant une dimension des pores qui se trouve dans la plage de 0,4 à 0,6 µm pour donner lieu à un coefficient de tamisage du filtre inférieur à 0,4 pour l'Immunoglobuline M et supérieur à 0,6 pour l'albumine pour laisser passer des médiateurs de l'inflammatoires à fort poids moléculaire et b) un moyen destiné à retenir lesdits médiateurs, mais sans retenir la sérumalbumine, comprenant au moins une cartouche comprenant un matériau sorbant sélectionné du groupe consistant en une résine de polystyrène hydrophobe, une résine de polystyrène échangeuse d'ions, une résine de silice liée sélectionnée du groupe consistant en des résines de silice avec des groupes fonctionnels à phases liées, ou leurs mélanges.

2. Kit selon la revendication 1, **caractérisé en ce que** ladite résine de polystyrène hydrophobe est sélectionnée du groupe constitué des résines de styrène-méthacrylate de méthyle et des résines de divinylbenzène-polystyrène copolymères.

3. Kit selon les revendications 1 ou 2, **caractérisé en ce que** ledit matériau sorbant a une dimension de granulés comprise entre 35 et 200 microns.

4. Kit selon les revendications 1 ou 2, **caractérisé en ce que** ledit matériau sorbant a une dimension des pores comprise entre 50 et 3000 Å.

5. Kit selon l'une quelconque des revendications 1 à 3, **caractérisé en ce que** ladite cartouche comprend une résine de polystyrène/divinylbenzène ayant une dimension des pores de 300 Å et une dimension des granulés allant de 35 à 120 microns.

6. Kit selon la revendication 5, **caractérisé en ce que** ladite cartouche comprend une résine de polystyrène/divinylbenzène ayant une dimension des granulés de 75-120 microns.

7. Kit selon l'une quelconque des revendications précédentes, **caractérisé en ce que** ledit moyen destiné à retenir des médiateurs inflammatoires comprend plus d'une cartouche, chaque cartouche comprenant un matériau adsorbant différent conçu pour retenir un médiateur inflammatoire ou plusieurs médiateurs inflammatoires différents, les médiateurs inflammatoires retenus par chaque cartouche étant différents entre eux.

8. Kit selon les revendications 1 à 6, **caractérisé en ce que** lesdits médiateurs inflammatoires sont sélectionnés du groupe constitué de VEGF, Kallikréine, myoglobine, protéine C réactive, cytokines, et chémokines, en particulier, IL1, IL6, IL8, IL12, IL18, facteur de nécrose tumorale, protéine 1 inflammatoire des macrophages, protéine chémotactique du monocyte.
